# EUROPEAN PATENT APPLICATION

(11) **EP 2 685 255 A2**
(43) Date of publication of application: **15.01.2014**
(21) Application number: 13175789.0
(22) Date of filing: 09.07.2013
(51) Int. Cl.: G01N 29/24, B06B 1/06, G01N 29/28

(54) **Transducer module including curved surface frame, ultrasonic probe including transducer module, and method of producing curved surface frame**

(30) Priority: 12.07.2012 KR 20120076014
(71) Applicant: Samsung Electronics Co., Ltd, Gyeonggi-do 443-742 (KR)
(72) Inventor: Kim, Young Il, Gyeonggi-do (KR); Kim, Bae Hyung, Gyeonggi-do (KR); Song, Jong Keun, Gyeonggi-do (KR); Lee, Seung Heun, Seoul (KR); Cho, Kyung Il, Seoul (KR)
(74) Representative: Grünecker, Kinkeldey, Stockmair & Schwanhäusser

(57) **Abstract**

A transducer module includes a curved surface frame which is formed from a flexible material in a curved shape and includes a front surface and a rear surface; a transducer which is disposed on the front surface; and a support frame which is mounted on the rear surface and supports the curved surface frame.

## Description

### BACKGROUND

### 1. Field

Apparatuses and methods consistent with exemplary embodiments relate to a transducer module in which a transducer is arranged, an ultrasonic probe, and a method of producing a frame to which the transducer is fixed.

### 2. Description of the Related Art

A transducer serves to convert a particular type of energy into another type of energy, and is also referred to as a converter. A transducer may be used in various devices, such as antennas, speakers, earphones, cathode ray tubes (CRTs), sonar detectors, and various sensors, and converts an electrical signal, an operation, a variety of waves such as a sound wave and an electromagnetic wave, and the like into a predetermined electrical signal, light, an image signal, and the like. The transducer is used to generate an ultrasonic wave in an ultrasonic probe of an ultrasonic diagnostic device, which is used to observe and diagnose the internal areas of a human body.

The ultrasonic diagnostic device of the related art applies a predetermined alternating current (AC) to each ultrasonic transducer mounted to the ultrasonic probe to create a predetermined vibration at the ultrasonic transducer, with an ultrasonic wave being generated by the vibration. The generated ultrasonic wave is irradiated into an object, for example, a human body, and is then reflected and returned from the various internal organs of the human body. Accordingly, the internal areas of the human body may be observed and diagnosed, by receiving the reflected ultrasonic wave with the ultrasonic transducer, converting the received ultrasonic wave into an electrical signal, and creating an image in response to the electrical signal.

In the related art ultrasonic diagnostic device, the ultrasonic transducers are arranged on a two-dimensional (2D) planar substrate disposed inside or outside a housing of the ultrasonic probe, and a curved or semicircular lens covers the ultrasonic transducers and the substrate. That is, the ultrasonic transducers are uniformly arranged within the lens in a 2D plane. However, there is a problem of constructing a beamforming algorithm for focusing the ultrasonic wave due to an outer shape and internal medium of the lens, and having a complicated system configuration in connection with the same. Accordingly, beamforming efficiency may be deteriorated.

On the other hand, if curvature of the lens is minimized to reduce system complexity, an area diagnosable by the ultrasonic diagnostic device may be limited. Accordingly, it may be cumbersome to image and observe a wide area of the object.

### SUMMARY

Exemplary embodiments may address at least the above problems and/or disadvantages and other disadvantages not described above. Also, the exemplary embodiments are not required to overcome the disadvantages described above, and an exemplary embodiment may not overcome any of the problems described above.

Therefore, exemplary embodiments provide a transducer module in which a transducer may be arranged on a curved surface frame, an ultrasonic probe using the transducer module, and a process of producing the curved surface frame used in the transducer module.

Specifically, various quantities of information are more efficiently collected compared to the related art, by arranging a transducer on a 3D curved surface frame having a variety of forms.

The transducer may be arranged in various forms compared to being arranged on a 2D plane of the related art to thereby sense and observe a particular type of energy such as an ultrasonic wave in more detail in a wider range, with the consequence that the performance of various devices using the transducer may be improved and enhanced.

In addition, manufacturing convenience and efficiency of various devices using a transducer may be promoted by simplifying configurations of the various devices using the transducer, for example, an ultrasonic probe, or algorithm complexity for beamforming, as the transducer is arranged in a wider variety of forms.

Furthermore, an ultrasonic wave may be easily irradiated in various angles and directions and easily received and collected from various directions by arranging the transducer on a 3D curved surface, instead of arranging a transducer on a 2D plane as in the apparatus of the related art.

According to one or more of exemplary embodiments, there are provided a transducer module including a curved surface frame on which a transducer is arranged, a method of producing the curved surface frame, and an ultrasonic probe including a transducer module as an example to which the transducer module including the curved surface frame is applied.

In accordance with an aspect of an exemplary embodiment, a transducer module includes at least one transducer, a curved surface frame which is formed in a curved shape on a front surface thereof and in which the transducer is disposed on the front surface, and a support frame which is mounted on a rear surface of the curved surface frame and supports the curved surface frame. The transducer module may include a lens attached on the front surface on which the transducer is disposed.

The transducer may include a magnetostrictive ultrasonic transducer, a piezoelectric ultrasonic transducer, or a capacitive micromachined ultrasonic transducer (cMUT), and these transducers may be combined and used as applicable.

The curved surface frame may be formed in a convex or concave shape in a direction of the front surface, and be made of a material having flexibility over a certain level. Specifically, the material having flexibility may include silicon, silicon oxide, quartz, glass, polymer, various metals, and the like.

The curved surface frame may be formed, on the front surface thereof, with an embossment or intaglio pattern such as a seating groove in which the transducer is disposed, and thus the transducer may be arranged in a regular pattern. A groove for tiling corresponding to the curved surface frame may be formed so that the transducer is tiled on the front surface.

The curved surface frame may be manufactured by a method of producing a curved surface frame.

In accordance with an aspect of an exemplary embodiment, a method of producing a curved surface frame includes, for example, polishing or cutting a back surface of a master mold to acquire the master mold having flexibility such that the master mold which is formed, on a front surface thereof, with an embossment or intaglio pattern has a thickness corresponding to a standard range, pressing a curved surface frame material against the master mold to form a predetermined intaglio or embossment pattern on the curved surface frame material, bending the mutually pressed master mold and curved surface frame material together, and separating the master mold and curved surface frame material, which are bent together, from each other. As a result, the curved surface frame may be finally produced which is formed with a predetermined intaglio or embossment pattern and is formed in a curved shape having predetermined curvature.

In accordance with an aspect of an exemplary embodiment, a method of producing a curved surface frame includes, for example, polishing or cutting a back surface of a master mold to acquire the master mold having flexibility such that the master mold which is formed, on a front surface thereof, with an embossment or intaglio pattern has a thickness corresponding to a standard range, bending the master mold to acquire a curved master mold, pressing a curved surface frame material against the curved master mold, and separating the curved master mold and the curved surface frame material from each other.

The master mold may be any one of silicon, silicon oxide, quartz, glass, polymer, and metal having flexibility, and the curved surface frame material may be any one of silicon, silicon oxide, quartz, glass, polymer, and metal having flexibility.

In exemplary methods of producing the curved surface frame, the support frame may be a curved substrate or a flexible substrate to control an electrical signal applied to the transducer.

According to one or more of exemplary embodiments, the transducer module may include a curved surface frame which is formed in a semicircular shape, in which at least one transducer is disposed on a front surface thereof, and which is made of a flexible material, and a support frame which is mounted on a rear surface of the curved surface frame and supports the curved surface frame. The transducer module may include a curved surface frame which is formed in a convex or concave curved shape in a direction of a front surface thereof, in which at least one transducer is disposed on the front surface, and which is made of a flexible material, and a support frame which is mounted on a rear surface of the curved surface frame and supports the curved surface frame.

In accordance with an aspect of an exemplary embodiment, an ultrasonic probe including the transducer module includes at least one ultrasonic transducer, a curved surface frame which is formed in a curved shape on a front surface thereof, in which the ultrasonic transducer is disposed on the front surface, and which is made of a flexible material, a support frame which is mounted on a rear surface of the curved surface frame to support the curved surface frame and is formed with a circuit to control an electrical signal applied to the transducer, and a lens which is mounted on the front surface of the curved surface frame.

The curved surface frame may be formed in a convex or concave shape in a direction of the front surface, and be made of a flexible material. The flexible material may be at least one selected from the group consisting of silicon, silicon oxide, quartz, glass, polymer, and metal.

The curved surface frame may be formed, on the front surface thereof, with an embossment or intaglio pattern such as a seating groove in which the transducer is disposed. Accordingly, the transducer may be exactly and easily disposed. The transducer may be tiled and disposed on the front surface of the curved surface frame.

The support frame may be a substrate having a curved shape, for example, a curved substrate or a flexible substrate.

A transducer may be a magnetostrictive ultrasonic transducer, a piezoelectric ultrasonic transducer, or a cMUT (capacitive micromachine ultrasonic transducer).

### BRIEF DESCRIPTION OF THE DRAWINGS

The above and/or other aspects will become more apparent by describing certain exemplary embodiments, with reference to the accompanying drawings, in which:
FIG. 1 is a perspective view illustrating a transducer module according to an exemplary embodiment;
FIG. 2 is a cross-sectional view illustrating the transducer module according to an exemplary embodiment;
FIGS. 3A, 3B, and 3C are perspective views illustrating examples of the transducer module according to an exemplary embodiment;
FIG. 4 is a view of a transducer arranged in the transducer module according to an exemplary embodiment;
FIG. 5 is a flowchart illustrating a process of producing a curved surface frame according to an exemplary embodiment;
FIG. 6A, 6B, 6C, 6D, and 6E are cross-sectional views of the process of producing the curved surface frame according to an exemplary embodiment;
FIG. 7 is a flowchart illustrating a process of producing a curved surface frame according to an exemplary embodiment;
FIG. 8 is perspective view illustrating an ultrasonic probe according to an exemplary embodiment; and
FIG. 9 is a cross-sectional view illustrating the ultrasonic probe according to an exemplary embodiment.

### DETAILED DESCRIPTION

Certain exemplary embodiments are described in greater detail below with reference to the accompanying drawings.

In the following description, the same drawing reference numerals are used for the same elements even in different drawings. The matters defined in the description, such as detailed construction and elements, are provided to assist in a comprehensive understanding of exemplary embodiments. Thus, it is apparent that exemplary embodiments can be carried out without those specifically defined matters. Also, wen-known functions or constructions are not described in detail since they would obscure exemplary embodiments with unnecessary detail.

Even though exemplary embodiments describe an ultrasonic transducer in detail, the present concept is not limited to the ultrasonic transducer, and other suitable transducer may be used depending on applicable fields and technologies by using the transducer module described below.

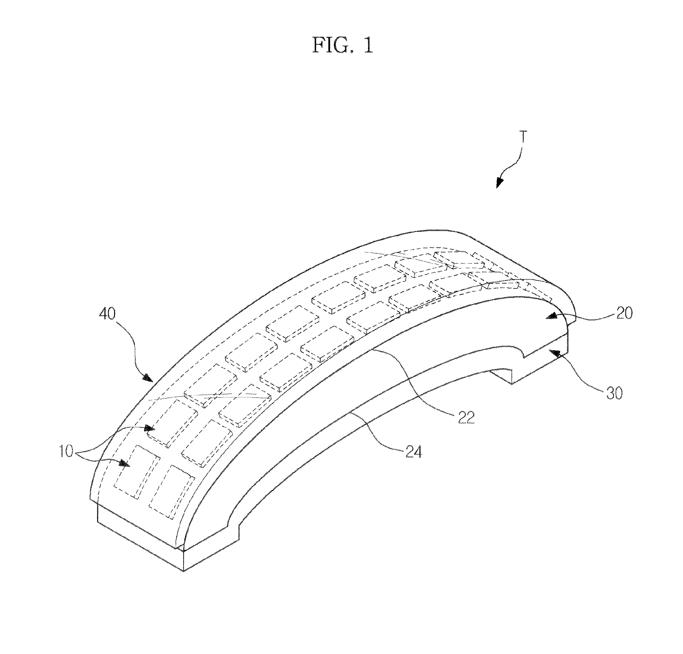

A transducer module according to an exemplary embodiment is described below with reference to FIGS. 1 to 4.

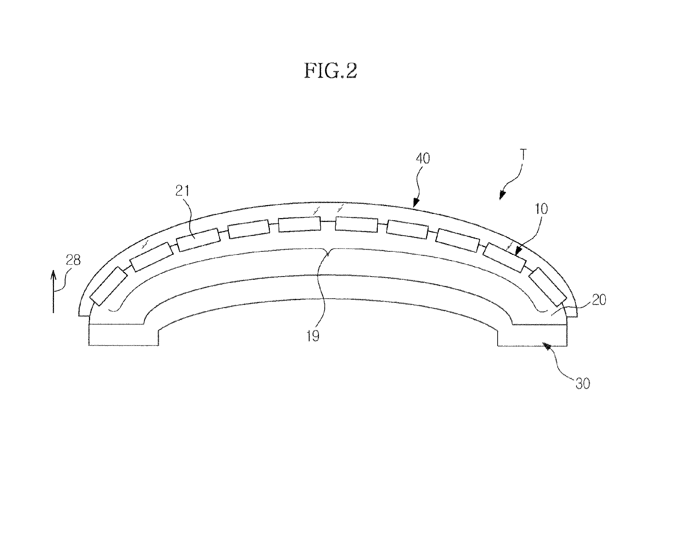

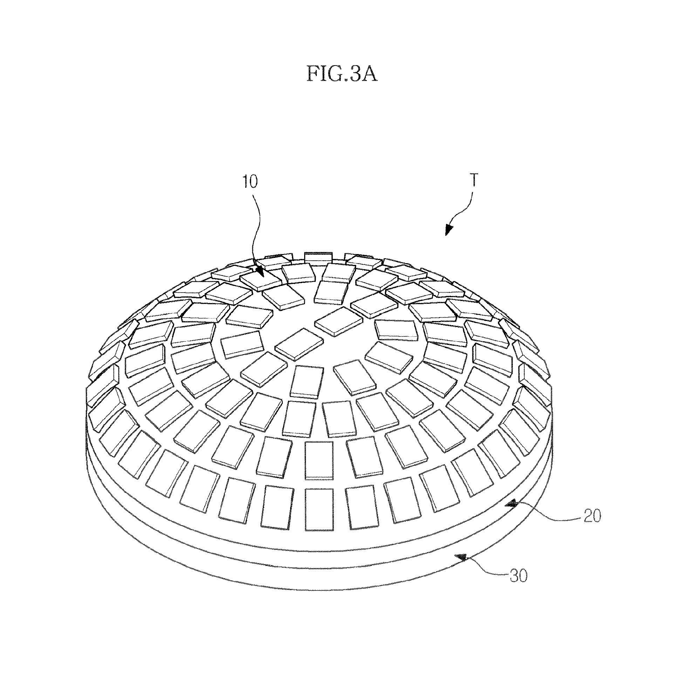

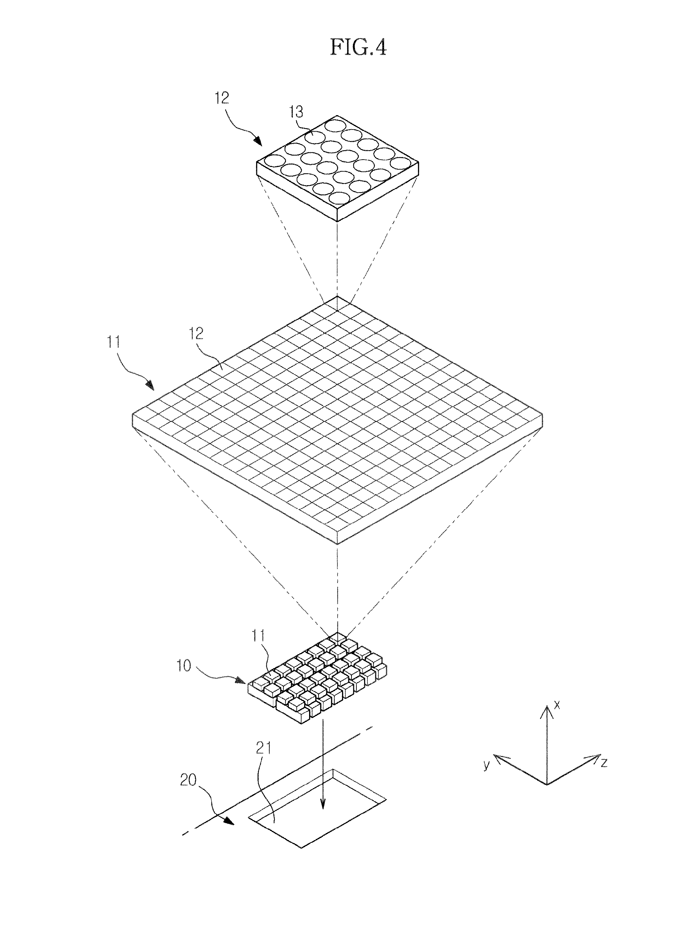

FIG. 1 is a perspective view illustrating a transducer module according to an exemplary embodiment. FIG. 2 is a cross-sectional view. FIGS. 3A to 3C are perspective views illustrating examples of the transducer module. FIG. 4 is a view of a transducer, according to an exemplary embodiment, which is arranged in the transducer module.

As shown in FIGS. 1 and 2, the transducer module T according to an exemplary embodiment may include one or more transducers 10, a curved surface frame 20 on which the transducer 10 is arranged, and a support frame 30 which is attached and mounted to the curved surface frame 20.

The transducer module T may further include a lens 40 which covers all or a portion of the curved surface frame 20.

The transducer 10 converts a particular type of energy into another type of energy to provide a function for an applicable technical field, for example, a function of creating or receiving an ultrasonic wave. For example, the transducer 10 performs a function of converting an electrical signal or the like into a sound wave, an ultrasonic wave, a pressure, or the like, or conversely, a function of converting a sound wave, an ultrasonic wave, a pressure, or the like into an electrical signal or the like. According to technical field, device, or use to which the transducer module T is applied, a transducer which is suitable for each function may be used as the transducer 10.

An example in which the transducer module T is applied to the ultrasonic probe is described below in more detail. The transducer 10 may be an ultrasonic transducer.

The ultrasonic transducer is a transducer which converts AC energy having a predetermined frequency into mechanical vibration having the same frequency.

The ultrasonic transducer operates as follows. When AC power, which is supplied from an external mechanical device, an internal battery, or the like, is applied to the transducer 10, a piezoelectric vibrator, a thin film, or the like of the transducer 10 vibrates to create an ultrasonic wave, and an external object is irradiated by the created ultrasonic wave. The irradiated ultrasonic wave is reflected from targets having different depths within the object, and the transducer 10 receives an ultrasonic echo signal, which is converted into an electrical signal. The converted electrical signal is processed by a processor, and thus an image with respect to an inner structure of the object is created and displayed on a monitor for a view by a user.

In an exemplary embodiment, the ultrasonic transducer 10 may include a magnetostrictive ultrasonic transducer, which uses a magnetostrictive effect of a magnetic substance, a piezoelectric ultrasonic transducer which uses a piezoelectric effect of a piezoelectric substance, a cMUT which transmits and receives an ultrasonic wave using vibrations of several hundred or thousands of micromachined thin films, or any other appropriate ultrasonic transducer.

The curved surface frame 20 according to an exemplary embodiment is described below in detail.

In an exemplary embodiment, the curved surface frame 20 is a frame on which the transducers 10 are arranged, and which fixes and supports the transducers 10. The curved surface frame 20 has a 3D shape which is formed in a uniform curved shape at a front surface 22, and the transducers 10 may be arranged in the form of a curved surface having a predetermined curvature.

According to an exemplary embodiment, the curved surface frame 20 may be formed in various shapes, as shown in FIGS. 2 to 3C, and may allow at least one transducer 10 to be arranged in the 3D shape, and not in a 2D plane of the related art. Consequently, the ultrasonic wave, for example, generated by the transducer may be irradiated in a given direction, and beamforming may be easily performed with respect to the ultrasonic wave, which is received from various directions, without beamforming algorithm complexity.

In an exemplary embodiment, the curved surface frame 20 may be made of a smooth and relatively flexible material to be easily bent or may be made of a material having relatively less flexibility. However, when the curved surface frame 20 is made of a material having flexibility over a certain level, the curved surface frame 20 may be easily produced and deformed in shape. Accordingly, the curved surface frame 20 may be made of a material having flexibility over a certain level.

The material having flexibility over a certain level is a material which may be easily bent by force of a person or a machine, for example, when being produced or immediately after being produced through a master mold, as described in greater detail below. As another example, a material, which may be hardened through certain processing after the above-described process, may be used as the material of the curved surface frame 20.

For example, the curved surface frame 20 may be made of a flexible material such as silicon, silicon oxide, quartz, glass, polymer, or various flexible metals. Each of these materials has a certain level of flexibility through predetermined processing, and is a material whose surface may be formed with a predetermined pattern through an imprinting method.

According to an exemplary embodiment, the curved surface frame 20 may be made of only one of the above-described materials, of a combination of a plurality of materials, for example, using a plurality of materials which overlaps to form a plurality of layers, or of separate materials depending on respective regions of the curved surface frame 20. This may be determined depending on a purpose for which the transducer module T is used.

The curved surface frame 20 made of a flexible material may have various forms and shapes.

For example, in an exemplary embodiment, the curved surface frame 20 may have an outer shape, which is bent in a first direction 28 extending from the rear surface 24 toward the front surface 22 of curved surface frame 20, and convexly protrudes in the first direction 28, as shown in FIG. 2. The curved surface frame 20 may have a curved surface having a fixed curvature value, may have a curved surface having a uniformly varying curvature value or curvature values different from each other according to positions on the curved surface, or may have a curved surface having an irregular curvature value as applicable. That is, according to technical field, purpose, or use to which the transducer module T is applied, an optimal curvature value or curvature function may be selected to correspond to the use, the purpose, or the like.

When the curved surface frame 20 is formed to have the curved surface, the transducers 10 may irradiate, for example, an ultrasonic wave or a sound wave in a wider range as compared to the related art. In addition, it is possible to easily perform beamforming with respect to an energy source, for example, a reflection echo of the ultrasonic wave or the like, which is collected using pre-given information with respect to the convex-shaped curvature, without the beamforming algorithm complexity.

In an exemplary embodiment, the curved surface frame 20 may be formed in a semicircular shape, as shown in FIG. 3A, and the transducers 10 may be arranged in a semicircular shape. Thus, the ultrasonic wave generated by each transducer 10 may irradiate even a wider range compared to the convex-shaped curved surface frame 20 shown in FIG. 2, and may receive the returned reflection echo in a wider area. The lens 40 may be attached on the side at which the transducer 10 of the curved surface frame 20 is disposed, as shown in FIG. 2.

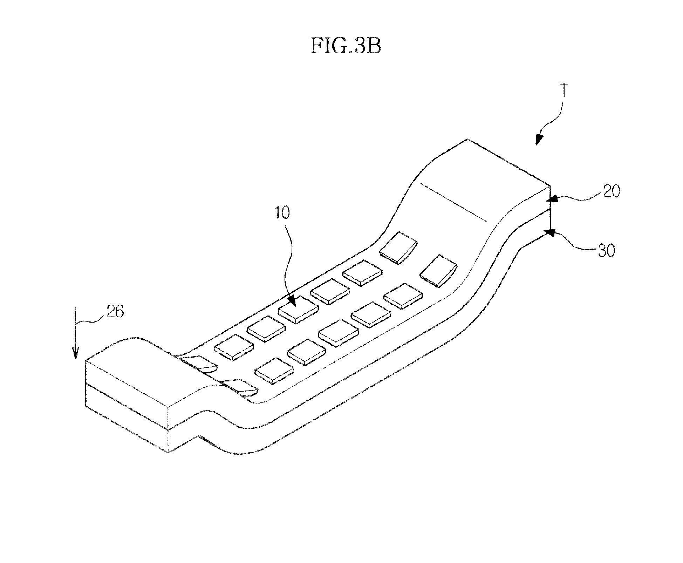

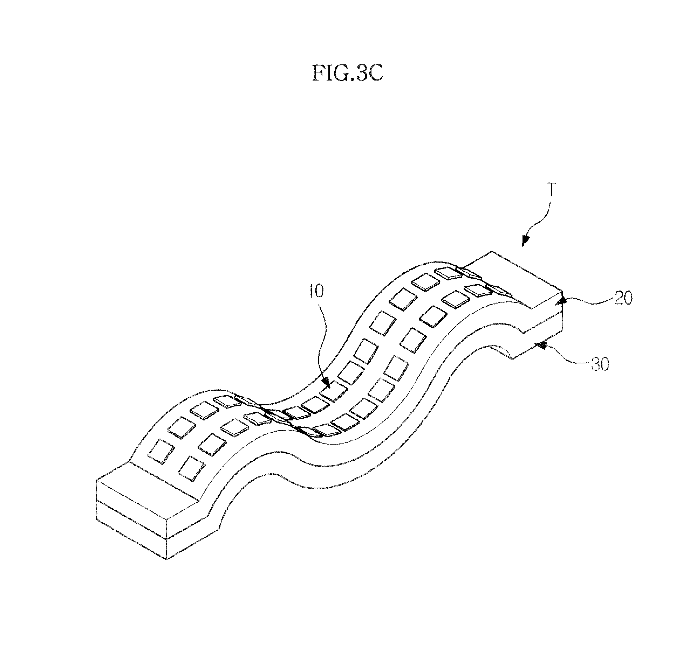

In an exemplary embodiment, the curved surface frame 20 may be formed in a concave shape, which is bent in a second direction 26 extending from the front surface 22 toward the rear surface 24 of curved surface frame 20, opposite the first direction 28, and is concavely recessed in a direction 26, as shown in FIG. 3B. For example, as shown in FIG. 3C, the curved surface frame 20 may have a wave shape in which the convex shape and the concave shape are combined. Accordingly, an optimal shape may be selected and applied to the curved surface frame 20, depending on field, technology, or use to which an exemplary embodiment is applied.

The curved surface frame 20 according to an exemplary embodiment may be produced in various corresponding shapes so that the transducer 10 may be arranged in various forms depending on the use. Therefore, the curved surface frame 20 may have various shapes such as a bell shape or a spindle shape, as well as the shapes shown in FIGS. 2 to 3C.

Accordingly, using the curved surface frame 20 suitable for field to which the transducer module T is applied, for example, suitable for the object to be measured by the ultrasonic probe, a more precise and clear image may be obtained. Consequently, an optimized diagnosis and test may be possible.

When the curved surface frame 20 according to an exemplary embodiment is made of a flexible material, it is possible to somewhat freely design an outer shape of the curved surface frame 20 to be suitable for technologies to which the transducer module T is applied, for example, depending on a sound output direction in a speaker or a shape of an object to be measured or tested. Therefore, the transducer module T may be applied to various technical fields, and a more precise and optimized result may be obtained.

The following description is given with respect to the mounting of the transducers 10 on the curved surface frame 20.

According to an exemplary embodiment, an auxiliary place unit may be formed on the curved surface frame 20 so that the transducers 10 may be arranged thereon. The auxiliary place unit may be, for example, a groove or grooves 21, as shown in FIG. 2, or a protrusion which protrudes in the form of embossment. The groove 21 or a protrusion may be arranged on the front surface 22 of the curved surface frame 20 in a regular pattern by forming an embossment or intaglio pattern 19 on the front surface 22 of the curved surface frame 20.

The embossment or intaglio pattern, for example, including the grooves 21, may exactly dispose each transducer 10 at a desired set position on the curved surface frame 20, and may allow the set position to be stably held.

The pattern formed on the curved surface frame 20 may include grooves for tiling so that the transducers 10 may be tiled on the front surface of the curved surface frame 20.

Each transducer 10 may be arranged according to the pattern formed on the curved surface frame 20 and needs to be fixed to the curved surface frame 20 to maintain the stability. According to an exemplary embodiment, an adhesive, such as an epoxy resin adhesive, is placed between the transducer 10 and the curved surface frame 20, to bond the transducer 10 to the curved surface frame 20. Of course, other coupling, fixing, or adhering, which may bond or fix the transducer 10 and the curved surface frame 20, may be used.

In an example described below with reference to FIG. 4, the transducer 10 is a cMUT array to generate an ultrasonic wave, which is disposed within the intaglio pattern of the curved surface frame 20. Referring to FIG. 4, the cMUT array may be a 2D array which is configured by a plurality of tiles 11 (for example, thirty two tiles), and one tile 11 may include elements 12 (for example, 256 elements in a 16 x 16 arrangement). Each of the elements 12 may include twenty thin films 13. The element generates an ultrasonic wave by vibrating in response to application of an electrical signal from, for example, the support frame 30 to be described below in detail.

Each cMUT array is bonded to the groove 21 of the intaglio pattern of the curved surface frame 20 using an epoxy resin adhesive or the like. As described above, the cMUT arrays may be directed in respective predetermined directions different from each other depending on the shape of the curved surface frame 20. Consequently, the cMUT arrays may irradiate the ultrasonic wave in various directions or receive the ultrasonic wave from various directions.

Although not illustrated the curved surface frame 20 may be formed with a wire which is connected to the transducers 10 and an external power source or a processor. According to an exemplary embodiment, the wire may be formed to pass through the inside of the curved surface frame 20. According to an exemplary embodiment, the wire may be connected along the outer surface of the curved surface frame 20. When the support frame 30 is a substrate having a curved surface of uniform curvature or a substrate to control an electrical signal applied to each transducer 10 as a flexible substrate, the wire is wired so that the transducer 10 may be connected to the substrate.

According to an exemplary embodiment, the curved surface frame 20 may be produced (a) by deforming a shape of a master mold, for example, by bending the master mold, and pressing a curved surface frame material to the master mold or (b) by pressing together the master mold and the curved surface frame material and deforming the master mold and the curved surface frame material together in the pressed state, as described below in more detail.

The support frame 30 supports the curved surface frame 20, and assumes a shape of the curved surface frame 20, for example, the convex shape as shown in FIG. 2. The support frame 30 may be mounted on a rear surface 24 of the curved surface frame 20 on which the transducer 10 is not arranged, as shown in FIGS. 2 to 3C.

The support frame 30 may be formed in a predetermined bent shape. For example, the support frame 30 may be formed such that the curved surface frame 20 made of a flexible material may be held and supported in the predetermined shape. The support frame 30 may be made of a flexible material. However, the support frame 30 may be made of a material having relatively less flexibility or a more hardened material in order to support the curved surface frame 20, based on an application.

The support frame 30 may be bonded to the curved surface frame 20 using an epoxy resin adhesive or the like, or may be coupled using any other appropriate coupling or fixing.

In an exemplary embodiment, the support frame 30 may be a curved substrate or a flexible substrate to control an electrical signal applied to the transducer 10. For example, the support frame 30 may be formed with circuits for the control of the transducer 10, for example, the control of the electrical signal applied to the transducer 10, conversion of energy by the transducer 10, and/or generation of an ultrasonic wave caused by conversion of the electrical signal.

According to an exemplary embodiment, the lens 40 is attached on and coupled to the front surface of the curved surface frame 20 on which the transducer 10 is arranged. The lens 40 protects the transducer 10 and the curved surface frame 20 by covering the curved surface frame 20 and the transducer 10 and blocking the same from the external elements, and focuses light energy, wave energy, or the like. In an exemplary embodiment, when the transducer module T is used in the ultrasonic probe, the lens 40 may be an acoustic lens.

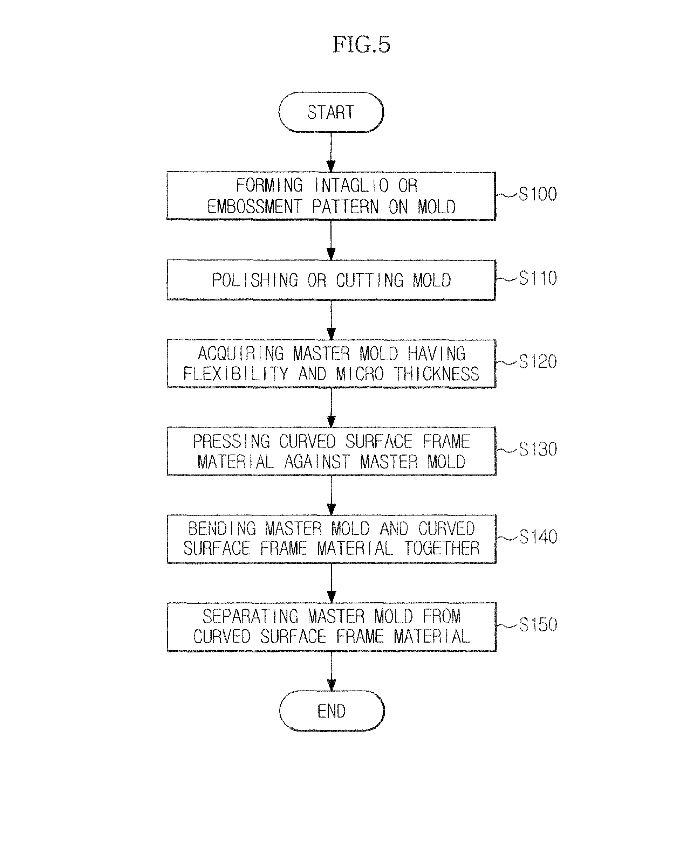

A method of producing a curved surface frame applied to the transducer module according to an exemplary embodiment is described below with reference to FIGS. 5 to 7.

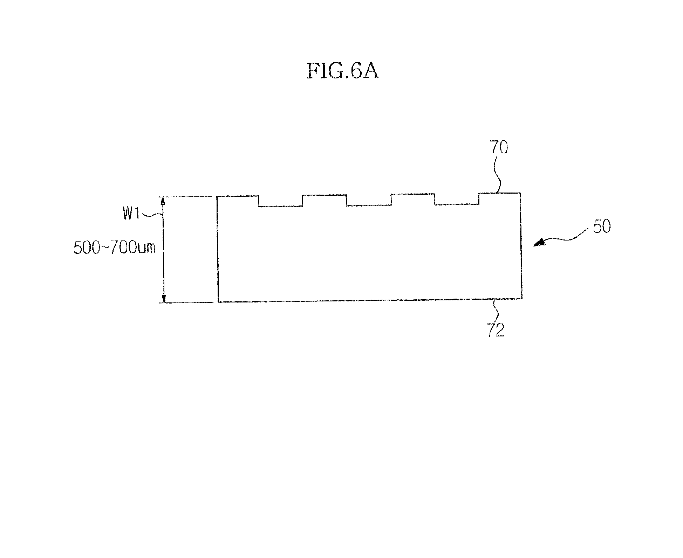

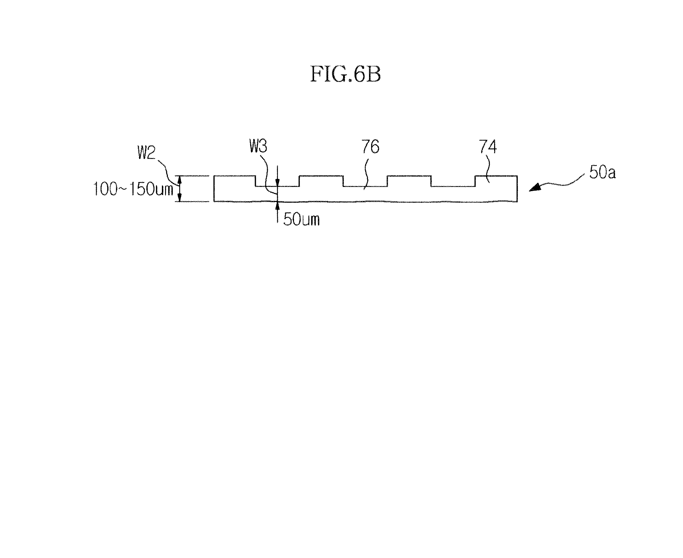

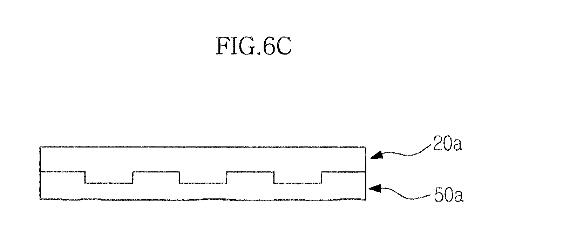

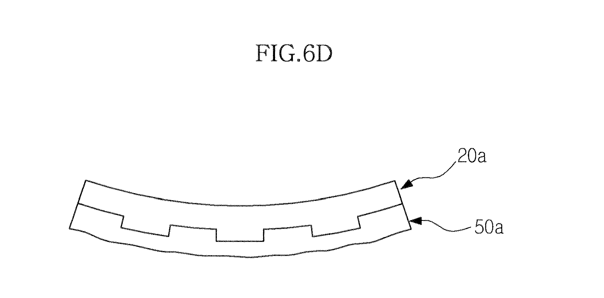

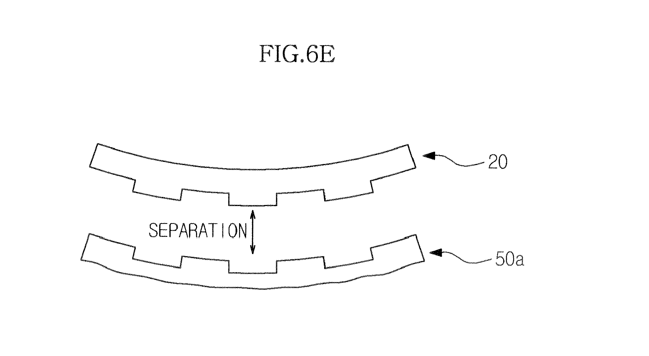

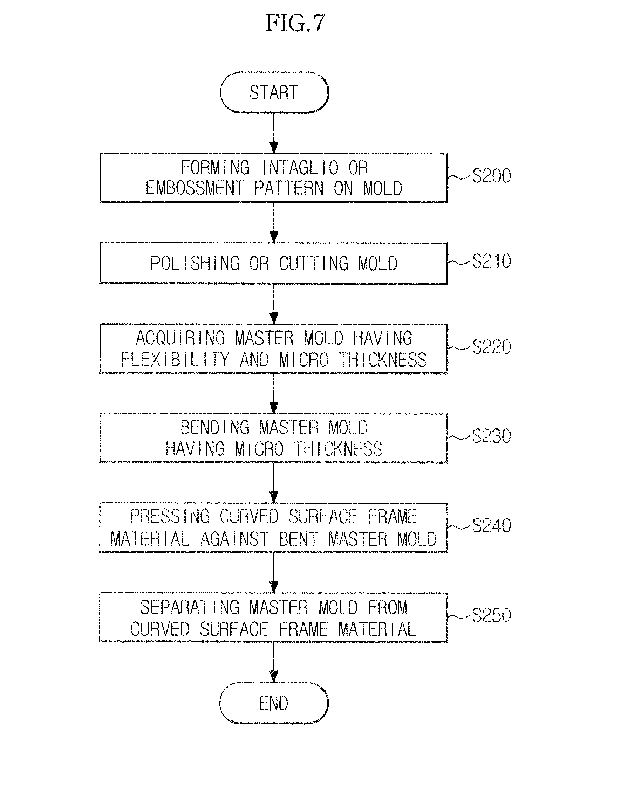

FIGS. 5 and 7 are flowcharts illustrating a process of producing a curved surface frame. FIG. 6 is a cross-sectional view for explaining the process of producing the curved surface frame.

A method of producing the curved surface frame according to an exemplary embodiment includes polishing or cutting a back surface of a mold to acquire the master mold having flexibility, forming on a front surface thereof an embossment or intaglio pattern, pressing a curved surface frame material against the master mold to form a predetermined intaglio or embossment pattern on the curved surface frame material, bending the mutually pressed master mold and curved surface frame material together, and separating the master mold from the curved surface frame material.

The method of producing the curved surface frame is described below in detail with reference to FIGS. 5 and 6.

As shown in FIG. 5, the curved surface frame 20 may be produced through the following six operations (1) to (6):
(1) an operation of forming an intaglio or embossment pattern on a mold 50 (operation S100);
(2) an operation of polishing or cutting the mold 50 (operation S110);
(3) an operation of acquiring a master mold 50a having flexibility and a micro thickness (operation S120);
(4) an operation of pressing a curved surface frame material 20a against the master mold 50a (operation S130);
(5) an operation of bending the master mold 50a and the curved surface frame material 20a together (operation S140); and
(6) an operation of separating the master mold 50a from the curved surface frame material 20a (operation S150).

In an exemplary embodiment, the master mold 50a used in the process of producing the curved surface frame for imprinting the curved surface frame, is made of a flexible material which may be easily bent. The flexible material for the mold may include silicon, silicon oxide, quartz, glass, polymer, various flexible metals, and the like. The mold may be made of the same material as the curved surface frame 20, or may be made of other material.

The process of producing the curved surface frame of the transducer module is described in more detail with reference to FIG. 6.

As shown in FIG. 6A, the mold 50 is formed with a pattern, for example, the intaglio or embossment pattern in which the transducer 10 is to be disposed on the curved surface frame 20. The pattern is formed to follow an array arrangement of the transducer 10, on a front surface 70 of the mold 50. The mold 50 may have a thickness w1 of approximately 500 to 700 µm (operation S100 and FIG. 6(A)).

Subsequently, the master mold 50a having a micro thickness is acquired by polishing or cutting another side on which the pattern is not formed, for example, the back surface 72 of the mold 50 to produce a thin master mold 50a with a thickness corresponding to a predetermined standard range (operations S110 and S120 and FIG. 6(B)). In the master mold 50a having a thin thickness, a first portion 74 protruding as an embossment is the thicker portion with a thickness w2 of approximately 100 to 150 µm. A second portion 76 recessed as an intaglio is the thinner portion with a thickness w3 of approximately 50 µm. As such, the master mold 50a is thinly polished or cut, and may be sufficiently flexed and smoothly bent.

The curved surface frame material is formed with a predetermined intaglio or embossment pattern corresponding to the embossment or intaglio pattern of the master mold 50a by pressing the curved surface frame material 20a against the master mold 50a. In an exemplary embodiment, the curved surface frame material 20a may be at least one of silicon, silicon oxide, quartz, glass, polymer, and flexible metal (operation S130 and FIG. 6(C)).

The master mold 50a and the curved surface frame material 20a are processed to have a uniformly bent shape as shown in FIG. 6(D) by pressurizing both the master mold 50a and curved surface frame material 20a together. For example, the master mold 50a and the curved surface frame material 20a are bent to form the curved surface frame 20 having the shape as shown in FIGS. 2 to 3C (operation S140).

Finally, the curved surface frame 20 having a desired shape, as for example shown in FIGS. 2 to 3C, is obtained by separating the master mold 50a from the curved surface frame material 20a (operation S150 and FIG. 6(E)).

Consequently, the support frame 30 is mounted to one surface of the acquired curved surface frame 20 while the transducer 10 is bonded to the other surface, thereby obtaining the transducer module T.

Alternatively, the curved surface frame 20 may be produced by a method different from the above, as follows.

A method of producing the curved surface frame 20 according to another exemplary embodiment may include polishing or cutting a back surface of a mold to acquire the master mold having flexibility, forming on a front surface thereof an embossment or intaglio pattern, bending the master mold to acquire a curved master mold, pressing a curved surface frame material against the curved master mold, and separating the curved master mold from the curved surface frame material.

This method is described in more detail with reference to FIG. 7. The method of producing the curved surface frame 20 according to an exemplary embodiment may include the following six operations (a) to (f):
(a) an operation of forming an intaglio or embossment pattern on a mold 50 (operation S200);
(b) an operation of polishing or cutting the mold 50 (operation S210);
(c) an operation of acquiring a master mold 50a having flexibility and a micro thickness (operation S220);
(d) an operation of bending the master mold 50a having a micro thickness (operation S230);
(e) an operation of pressing a curved surface frame material 20a against the bent master mold 50a (operation S240); and
(f) an operation of separating the master mold 50a from the curved surface frame material 20a (operation S250).

The operations (a) to (c) are substantially identical with the above-described operations (1) to (3) of FIG. 5.

Accordingly, the master mold 50a having the micro thickness is similarly acquired in operations (a) to (c) (operations S200 to S220).

The curved master mold 50a having a curvature corresponding to the curved shape of the curved surface frame to be produced is acquired by processing the master mold 50a to be bent in a certain shape without pressing the curved surface frame material 20a to the master mold 50a (operation S230).

The curved surface frame 20 having the curved shape, as shown in examples of FIGS. 2 to 3C, is obtained by mutually pressing the master mold 50a having a micro thickness and the above-described curved surface frame material 20a, and separating the master mold 50a from the curved surface frame material 20a (operations S240 and S250).

According to an exemplary embodiment, the curved surface frame 20 having a curved shape may be obtained by pressing the master mold 50a having a micro thickness and the curved surface frame material 20a together and then processing the same. However, according to another exemplary embodiment, the curved surface frame 20 having a curved shape may be obtained by pre-processing the master mold 50a to have a curved shape before pressing the master mold 50a and the curved surface frame material 20a.

The curved surface frame 20 may be obtained by the above-described methods, but this is not limiting. The curved surface frame 20 may be produced using other methods as applicable. However, it should be understood that the curved surface frame 20 according to an exemplary embodiment is not limited to being produced by the above-described methods.

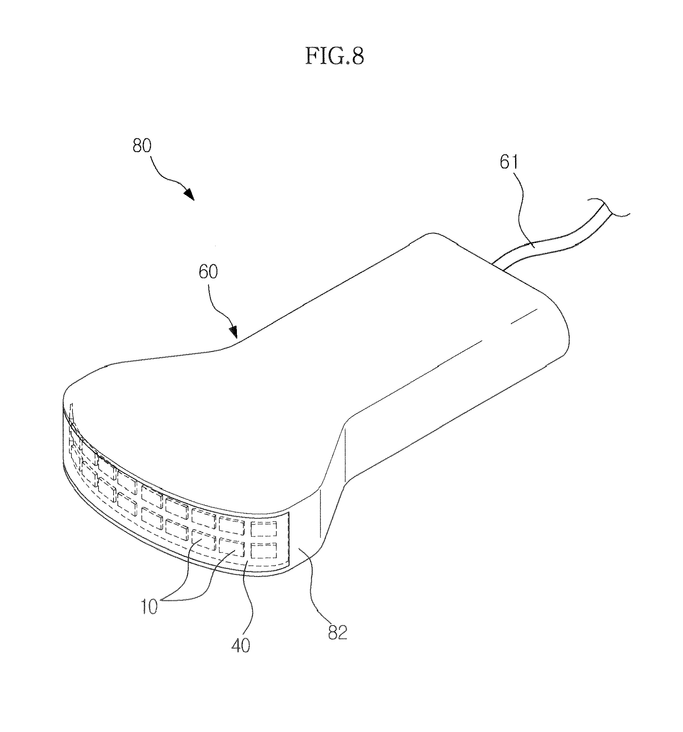

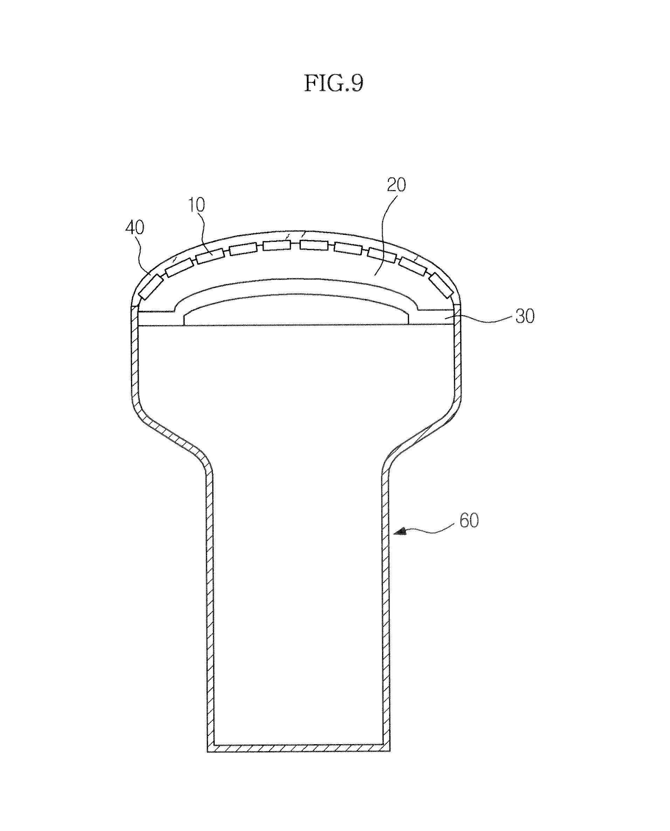

An ultrasonic probe of an exemplary embodiment to which the transducer module is applied is described with reference to FIGS. 8 and 9.

FIGS. 8 and 9 are a perspective view and a cross-sectional view illustrating an ultrasonic probe according to an exemplary embodiment.

In an exemplary embodiment, the transducer module T may be used in the ultrasonic probe 80 of the ultrasonic diagnostic device.

In more detail, in an exemplary embodiment, the ultrasonic probe may include one or more ultrasonic transducers 10, a curved surface frame 20 which is formed in a curved shape on a front surface 82 on which the ultrasonic transducer 10 is disposed, and which is made of a flexible material, and a support frame 30 which is mounted on a rear surface of the curved surface frame 20 to support the curved surface frame, and is formed with a circuit to control an electrical signal applied to the transducer. The ultrasonic probe may further include a lens 40 which is mounted on the front surface 82 of the curved surface frame, as shown in FIGS. 8 and 9.

In an exemplary embodiment, the ultrasonic probe may include a probe housing 60 to which the support frame 30 is attached or the support frame 30 and the curved surface frame 20 are attached, or within which the support frame 30 or a portion of the support frame 30 and curved surface frame 20 are accommodated to be fixed, as shown in FIG. 8.

The transducer 10 is an ultrasonic transducer which irradiates an ultrasonic wave into an object and receives a reflection echo from the object and may include a magnetostrictive ultrasonic transducer, a piezoelectric ultrasonic transducer, a cMUT, or the like.

The curved surface frame 20 may be formed in a convex shape, a concave shape, a semicircular shape, or a wave shape, or may have various shapes depending on properties of the object to be irradiated by the ultrasonic wave. The curved surface frame 20 may be formed with a pattern in which the transducer 10 may be exactly disposed, as for example, an embossment pattern or an intaglio pattern. A plurality of transducers 10 may be arranged on the curved surface frame 20 as a tile.

A flexible substrate is used as the support frame 30, to control generation of the ultrasonic wave by applying a predetermined power to the transducer 10.

The housing 60 is directly attached to the support frame 30 using an adhesive or is bonded to the support frame 30 and the curved surface frame 20. The housing 60 may stably fix the transducer module T, and a variety of other modules, for example, heat radiation modules or various circuits for operation of the probe device, may be mounted inside the housing 60. Although not shown in the drawings, a handle or the like may be formed at an outer side of the housing 60 for the convenience of a user.

The ultrasonic probe 80 controls the ultrasonic transducer 10 using a signal generated from a circuit of the support frame 30 and generates an ultrasonic wave. The ultrasonic probe receives the reflection echo of the ultrasonic wave, which is reflected from the object, through the ultrasonic transducer 10. The received ultrasonic wave is converted into an electrical signal, and the electrical signal is transmitted to an external information processing unit, which includes an image processing portion creating an image from an electrical signal and the like, through a cable 61 or the like. A processor, which may perform the image processing, may be mounted on a circuit board of the support frame 30 within the housing 60.

In accordance with exemplary embodiments, the ultrasonic probe may allow irradiation of the ultrasonic wave in a wide area and collection of the reflection echo by enlarging an area in which the transducer module T is located at the tip of the probe, particularly the area at which the lens comes into contact with the object due to the presence of the curved surface frame 20. For example, the ultrasonic transducers are arranged in a large area, thereby receiving or irradiating the ultrasonic wave in a wider range, compared to the related art probe. Accordingly, a large-area ultrasonic system may be configured, and thus a region wider than the region of the related art device may be directly observed. Also, a large quantity of information may be further acquired.

Due to such acquisition of a large quantity of information, a more precise and clear image may be created compared to the image acquired by the related art probe. Moreover, when the outer shape of the curved surface frame 20 is designed to correspond to the shape of the object or an organ to be imaged, more precise and optimized information may be collected.

As described above, there are provided a transducer module including a curved surface frame on which a transducer is arranged, a method of producing the curved surface frame, and an ultrasonic probe including a transducer module as an example to which the transducer module including the curved surface frame is applied. Thus, it may be possible to simplify a configuration for manufacturing a variety of devices using the transducer, to facilitate a configuration of a beamforming system associated with the transducer, and to more efficiently collect various quantities of information through the transducer, as compared to the related art.

For example, it may be possible to facilitate the configurations of the various devices using the transducer, for example an ultrasonic probe of an ultrasonic diagnostic device, and to simplify algorithm complexity for beamforming, as the transducer is arranged in various 3D forms by deviating from a 2D arrangement of the related art. Consequently, the design of the beamforming system may be simplified and algorithm complexity may be avoided. Therefore, it may be possible to promote manufacturing convenience and efficiency of the various devices using the transducer.

In addition, it may be possible to more efficiently collect various quantities of information compared to the related art by arranging a transducer on a 3D curved surface frame having a variety of forms. For example, the transducer may be arranged in a wider variety of forms compared to being arranged on a 2D plane of the related art, thereby irradiating a particular type of energy such as an ultrasonic wave in a wider range, or sensing and observing energy within the wider range in more detail. Therefore, performance of various devices using the exemplary transducer may be improved and enhanced.

Although a few exemplary embodiments have been shown and described, exemplary embodiments are not limited thereto. It would be appreciated by those skilled in the art that changes may be made in these exemplary embodiments without departing from the principles of the disclosure, the scope of which is defined in the claims and their equivalents.

## Claims

1. A transducer module (T) comprising:
a curved surface frame (20) which is formed from a flexible material in a curved shape and comprises a front surface (22) and a rear surface (24);
a transducer (10) which is disposed on the front surface (22); and
a support frame (30) which is mounted on the rear surface (24) and supports the curved surface frame.

2. The transducer module according to claim 1, wherein the curved surface frame (20) is formed in a convex shape or a concave shape in a direction toward the front surface.

3. The transducer module according to claim 1, wherein the flexible material is at least one of silicon, silicon oxide, quartz, glass, polymer, and metal.

4. The transducer module according to claim 1, wherein the front surface (22) of the curved surface frame (20) comprises a groove (21), and
the transducer (10) is disposed in the groove.

5. The transducer module according to claim 1, wherein the transducer (10) is tiled and disposed on the front surface of the curved surface frame.

6. The transducer module according to claim 1, wherein:
the support frame (30) comprises a substrate configured to control an electrical signal applied to the transducer (10); and
the substrate comprises a curved substrate or a flexible substrate.

7. The transducer module according to claim 1, further comprising:
a plurality of ultrasonic transducers (10) disposed in a three-dimensional (3D) arrangement in the front surface (22) of the curved surface frame (20).

8. An ultrasonic probe (80) comprising:
a transducer module (T) according to one of the previous claims, wherein
the support frame (30) further comprises a circuit to control an electrical signal applied to the transducer (10) and a lense (40) which is mounted on the front surface (22).

9. A method of producing a curved surface frame (20) for a transducer module (T) configured to be disposed on a front surface (22) of the curved surface frame (20), the method comprising:
acquiring (S120, S220) a master mold (50a) by polishing (S110, S210) or cutting a back surface (72) of a thicker mold (50) formed of a flexible material;
forming (S100, S200) on a front surface (70) of the master mold (50a) a first embossment pattern or a first intaglio pattern;
forming a second intaglio pattern or a second embossment pattern on a surface frame material by one of (a) pressing (S130) the surface frame material against the front surface of the master mold and bending (S140) the mutually pressed master mold and curved surface frame material together or (b) acquiring a curved master mold by bending (S230) the master mold and pressing (S240) the surface frame material against the curved master mold; and
separating the master mold and the surface frame material.

10. The method according to claim 9, wherein the transducer module (T) comprises transducers(10),
the forming the second intaglio pattern or the second embossment pattern comprises forming grooves (21) on the surface frame material, and
the method further comprises fixing each of the transducers (10) in a corresponding groove(21).

11. The method according to claim 9, wherein the master mold (50a) comprises at least one of silicon, silicon oxide, quartz, glass, polymer, and flexible metal.

12. A method of producing a curved surface frame (20), the method comprising:
forming (S100, S200) a first embossment pattern or a first intaglio pattern on a first surface of a mold;
polishing (S110, S210) or cutting a second surface of the mold (50) to acquire a master mold (50a) having flexibility and a thickness corresponding to a given range, the second surface being opposite the first surface;
combining (S130, S140; S230, S240) a surface frame material with the master mold; and
acquiring (S150, S250) a curved surface frame by separating the master mold from the surface frame material.

13. The method according to claim 12, wherein the combining comprises:
pressing (S130) the surface frame material against the master mold to form a second intaglio pattern or a second embossment pattern on the surface frame material; and
bending (S140) the mutually pressed master mold and surface frame material together.

14. The method according to claim 12, wherein the combining comprises:
bending (S230) the master mold (50a) to acquire a curved master mold; and
pressing (S24) the surface frame material against the curved master mold.

15. The method according to claim 12, wherein the combining comprises:
pressing the surface frame material against the first surface; and
forming a second embossment pattern or a second intaglio pattern on the surface frame material, by pressing in the first intaglio pattern or the first embossment pattern, respectively.
